# DEMANDE DE BREVET EUROPEEN

(11) **EP 0 963 706 A1**
(43) Date de publication de la demande: **15.12.1999**
(21) Numéro de dépôt: 98201490.4
(22) Date de dépôt: 07.05.1998
(51) Int. Cl.: A23L 1/226, A23L 1/227

(54) **Mélange précurseur d'arôme**

(71) Demandeur: SOCIETE DES PRODUITS NESTLE S.A., 1800 Vevey (CH)
(72) Inventeur: Bel Rhlid, Rachid, 1066 Epalinges (CH); Chaintreau, Alain, 1228 Plan-Les-Ouates (CH); Pollien, Philippe, 1606 Forel/Lavaux (CH)
(74) Mandataire: Vuille, Roman

(57) **Abrégé**

Mélange précurseur d'arômes générant, lors d'une étape de chauffage, une note aromatique due à la formation de thiols.

## Description

La présente invention a pour objet un mélange précurseur d'arômes et son utilisation lors de la fabrication de compositions alimentaires.

Il est connu que les composés contenant du soufre sont des constituants importants dans le domaine des arômes alimentaires.

En effet, il est connu, dans "BACIS, VCF96, database of volatile compounds in food 1996" que les groupes thiols sont présents dans les arômes alimentaires, dégageant une odeur de rôti, une odeur de grillé formées lors de la cuisson de certaines compositions alimentaires, telles que, notamment, les viandes, les oeufs ou les végétaux, lors du procédé de fabrication de la bière ou lors du rôtissage du café, par exemple.

Malheureusement, les thiols sont des composés instables qui sont souvent perdus par évaporation ou par oxydation. Hofmann et al. dans J. Agric Food Chem., 1996, 44, 251-255, ont montré que les thiols sont oxydés en disulfures correspondants, après un stockage à une température de 6° C.

Par ailleurs, Mottram et al., dans J. Agric Food Chem., 1996, 44, 2349-2351, soutiennent que les groupements thiols peuvent être formés, en faisant réagir dans une solution aqueuse à chaud, des composés disulfures avec des protéines comprenant des groupes sulfhydryles, notamment des cystéines, ou des ponts disulfures, notamment des cystines. Dans ce document, ils mettent notamment en évidence le fait que la cuisson d'une solution aqueuse contenant un disulfure et de l'albumine permet la libération de groupes thiols, à un rendement de 44,8%.

Le but de la présente invention est de proposer un mélange précurseur d'arômes permettant de favoriser le dégagement d'une odeur de grillé ou d'une odeur de rôti précisément lors de la cuisson ou lors du réchauffage de compositions alimentaires.

A cet effet, la présente invention a pour objet un mélange précurseur d'arômes générant lors d'une étape de chauffage, une note aromatique due à la formation de thiols.

La présente invention a permis de mettre en évidence la nécessité de la présence de un ou plusieurs groupements sulfhydryles que l'on fait réagir avec un ou plusieurs polysulfures, de manière à permettre le dégagement d'une note aromatique.

Dans la suite de la description, on emploiera indifféremment l'expression "note aromatique" ou le terme "odeur" pour désigner la formation d'un arôme perceptible, sur le plan sensoriel, au niveau de l'odorat ou par voie retronasale.

De préférence, le mélange précurseur d'arômes est constitué d'un polysulfure et d'une source de soufre non volatile comprenant au moins un groupe sulfhydryle.

De préférence, le polysulfure est de type R-(S)ₙ-R' Où R et R' sont identiques ou différents et n est supérieur ou égal à 2. On peut notamment utiliser comme polysulfure un diméthyl disulfure, un bis(2-furfuryl)disulfure,un bis(3-méthyl-2-furanyl)disulfure, un bis(3-méthyl-2-butén-1-yl)disulfure ou un disulfure de thio-cétones, comme le bis-(2-oxopropyl) disulfure.

La présente invention concerne également un système aromatique dans lequel le polysulfure est obtenu par biotransformation d'un conjugué cystéine-aldéhyde à l'aide d'une levure boulangère dans un tampon phosphate à pH 7-9, de préférence à pH 8, contenant 10-30 mM de conjugué cystéine-aldéhyde.

La source de soufre non volatile, combinée ou non à d'autres constituants, peut être un acide aminé soufré, un peptide contenant au moins un acide aminé soufré, une protéine contenant au moins un acide aminé soufré et/ou un hydrolysat de protéines contenant au moins un acide aminé soufré, par exemple.
On peut utiliser comme source de soufre non volatile, selon la présente invention, de la cystéine, du glutathion, de l'albumine d'oeuf, du gluten, du BSA, des protéines de lait ou des hydrolysats de protéines de lait, par exemple.

De préférence on chauffe à pH neutre le mélange, selon l'invention, de manière à permettre la formation de thiols à un rendement de 48-90%.

La présente invention a également pour objet l'utilisation d'un tel mélange précurseur d'arômes dans la fabrication d'une composition alimentaire, de manière à favoriser le développement d'une note aromatique rôtie, grillée lorsqu'on chauffe la composition alimentaire en milieu aqueux ou à sec.

On peut notamment utiliser un tel mélange dans la fabrication de sauces, de soupes, de plats cuisinés réfrigérés ou congelés et/ou de compositions alimentaires lyophilisées, tels que des produits à base de café soluble, par exemple. On peut ajouter le mélange selon l'invention dans une proportion de 0,07-0,50% par rapport au poids sec de la composition alimentaire, par exemple.

On peut chauffer la composition alimentaire aux microondes, en milieu aqueux ou à sec, dans un four conventionnel et/ou dans de l'eau bouillante, par exemple. Il se dégage alors une odeur prononcée de rôti ou une odeur prononcée de grillé.

La présente invention a enfin pour objet un mélange précurseur d'arômes, dans lequel la source de soufre non volatile comporte une ou plusieurs protéines contenant au moins un acide aminé soufré, de manière à permettre la formation d'une poche hydrophobe emprisonnant un ou plusieurs polysulfures sous forme d'un complexe, et permettant ainsi de les protéger des altérations. On peut sécher ce complexe, de manière à obtenir une poudre aromatique stable.
On peut notamment le sécher par pulvérisation ou par lyophilisation, par exemple.

La présente invention est décrite plus en détails à l'aide de tests et d'exemples non limitatifs, ci-après. Dans ces tests et dans ces exemples, les pourcentages sont donnés en moles sauf indication contraire.

### Test 1: Réaction entre du bis(2-furfuryl) disulfide et une source protéique comprenant au moins un groupe sulfhydryle

On fait réagir, en milieu aqueux, à chaud, du bis(2-furfuryl) disulfure avec une source protéique, de manière à produire du 2-furfuryl-thiol isolé à l'aide d'un appareil de distillation-extraction simultanées, le SDE, à pression atmosphérique ou sous vide.

On calcule alors le rendement de formation de groupes thiols. Ce rendement est exprimé en pourcentage et est calculé sur la base de la quantité de groupes thiols qui devraient être formés, soit le double de la quantité molaire de disulfure.

L'ensemble de données est mentionné dans le tableau 1, ci-après.

**Tableau 1**

| | (a) | Source protéique | Rendement | mode d'isolation |
|---|---|---|---|---|
| témoin | | - | <1% | SDE/pression atmosphérique (100° C) |
| essai n°1 | 10 ppm | albumine d'oeuf | 87% | SDE/pression atmosphérique (100° C) |
| essai n°2 | 10 ppm | albumine d'oeuf dénaturée | 61% | SDE/pression atmosphérique (100° C) |
| essai n°3 | 10 ppm | albumine d'oeuf | <1% | SDE/sous vide (37° C) |
| essai n°4 | 100 ppm | gluten | 45% | SDE/pression atmosphérique (100° C) |
| essai n°5 | 100 ppm | BSA | 51% | SDE/pression atmosphérique (100° C) |
| essai n°6 | 100 ppm | β-lactoglobuline | 60% | SDE/pression atmosphérique (100° C) |
| essai n°7 | 1000 ppm | β-lactoglobuline + 1% de cystéine | 80% | SDE/pression atmosphérique (100° C) |
| essai n°8 | 100 ppm | hydrolysat de β-lactoglobuline | 20% | SDE/pression atmosphérique (100° C) |
| légende: (a) quantité de bis(2-furfuryl) disulfure / quantité de la source protéique | | | | |

Les résultats mentionnés au tableau 1 mettent en évidence le fait que la réaction, en milieu aqueux à chaud, entre un polysulfure, tel que le bis(2-furfuryl) disulfure, et une source protéique comprenant au moins un groupe sulfhydryle permet la formation de thiols correspondants, à un rendement intéressant.
Par ailleurs, l'essai n°3 permet de mettre en évidence le fait que si l'on fait réagir du bis(2-furfuryl) disulfure avec de l'albumine sous vide à température ambiante, aucun thiol correspondant n'est formé. Cela permet de confirmer le fait que le disulfure est complexé dans la poche hydrophobe formée par la protéine. On peut ainsi éviter la perte du polysulfure par évaporation et limiter la dispersion de son odeur avant le chauffage de la composition alimentaire à laquelle on a ajouté le mélange aromatique, selon l'invention.
Pour augmenter le rendement en thiol, on peut également former un complexe ternaire comprenant un disulfure, de la cystéine et une protéine, comme illustré à l'essai n°7.

### Test 2: Effet sur le rendement de la réaction de la quantité de polysulfure par rapport à la quantité de la source protéique contenant au moins un sulfhydryle

On calcule le rendement de la réaction en fonction de la quantité de polysulfure par rapport à la quantité de la source protéique contenant au moins un sulfhydryle.

Pour ce faire, on effectue plusieurs réactions, dans un milieu aqueux à chaud, avec, à chaque essai, une quantité croissante de bis(2-furfuryl)disulfure et une quantité constante d'albumine d'oeuf.
On calcule alors le rendement de formation de groupes thiols. Ce rendement est exprimé en pourcentage molaire et est calculé sur la base de la quantité de groupes thiols qui devraient être formés, soit le double de la quantité molaire de disulfure.

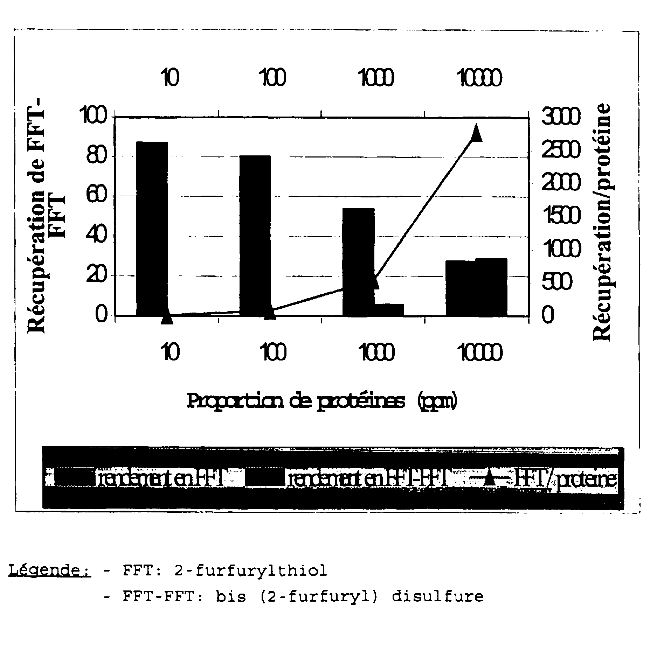

Les résultats sont mentionnés à la figure 1, ci-après.

La figure 1 met en évidence le fait que le taux de transformation du disulfure en funfurylthiol formé diminue quand le taux de bis(2-furfuryl)disulfure complexé dans la poche hydrophobe de la protéine augmente.

### Test 3: Réaction dans un milieu aqueux à chaud entre de l'albumine d'oeuf et différents polysulfures

On fait réagir, dans un milieu aqueux à chaud, de l'abumine d'oeuf avec différents polysulfures et l'on calcule le rendement de formation de thiol tel que décrit dans le test 1.

L'ensemble des résultats sont mentionnés dans le tableau 2, ci-dessous.

**Tableau 2**

| | polysulfure | (a) | rendement | méthode d'isolation |
|---|---|---|---|---|
| essai n°9 | bis(2-furfuryl) disulfure | 10 ppm | 87% | SDE/pression atmosphérique |
| essai n°10 | bis(3-méthyl-2-buten-1-yl) disulfure | 10 ppm | 30% | SDE/pression atmosphérique |
| essai n°11 | 2-furfurylthiol | 100 ppm | 61% | SDE/pression atmosphérique |
| essai n°12 | diméthyl trisulfure | 10 ppm | 7% | SDE/pression atmosphérique |
| légende: (a) quantité de polysulfure / quantité d'albumine d'oeuf. | | | | |

Les résultats mentionnés au tableau 2 mettent en évidence le fait que la réaction, en milieu aqueux à chaud, entre un polysulfure et une source protéique comprenant au moins un groupe sulfhydryle permet la formation de thiols correspondants.

Par ailleurs, ces résultats mettent en évidence le fait que cette réaction entre un polysulfure, le bis(2-furfuryl) disulfure, et une source protéique comprenant au moins un groupe sulfhydryle (essai n°9) permet la formation de thiols correspondants, à un rendement meilleur que dans le cas où l'on effectue une simple complexation entre un groupement thiol, le 2-furfurylthiol, et une source de protéines comprenant un groupement sulfhydryle (essai n°11). On peut suposer que cette différence de rendement est due au fait qu'il y a une réaction irréversible (essai n°11) entre le groupement thiol et la protéine avec formation de disulfures, des furfuryl-S-S-protéines, qui ne sont pas volatiles.
De plus, on n'a pas de perte de rendement lors de la réaction entre un polysulfure et une source protéique comprenant au moins un groupe sulfhydryle, car les thiols correspondants sont libérés au fur et à mesure de leur formation.

### Test 4: Acide aminé ou peptide comme source de soufre non volatile comprenant au moins un groupe sulfhydryle.

On fait réagir, dans un milieu aqueux à chaud, du bis(2-furfuryl)disulfure et un acide aminé ou un peptide, comme source de soufre non volatile comprenant au moins un groupe sulfhydryle. Le rapport massique bis(2-furfuryl) disulfure/source de soufre non volatile comprenant au moins un groupe sulfhydryle est de 1/10. On calcule alors le rendement de formation de thiol tel que décrit dans le test 1.

L'ensemble des résultats est mentionné dans le tableau 3, ci-dessous.

**Tableau 3**

| | (b) | rendemen t | valeur du pH |
|---|---|---|---|
| essai n°13 | cystéine | 10-59% | - |
| essai n°14 | cystéine | 87% | 7,6 |
| essai n°15 | glutathion | 80% | 7,6 |
| essai n°16 | cystine | 2,8% | 7,6 |
| Légende: (b) source de soufre non volatile contenant au moins un groupe sulfhydryle ou un pont disulfure. | | | |

Les résultats mentionnés au tableau 3 mettent en évidence le fait que le mélange de précurseur d'arômes selon la présente invention constitué d'un disulfure volatile, le bis(2-furfuryl)disulfure et d'un acide aminé soufré, la cystéine, ou d'un peptide contenant au moins un acide aminé soufré, le glutathion, permet la formation de thiols, lors d'une étape de chauffage en milieu aqueux, et ainsi génère une note aromatique.
Par contre, comme cela est illustré par l'essai n°16, si la source de soufre non volatile est une source de ponts disulfures, telle que la cystine, sans sulfhydryle libre, on obtient un rendement négligeable de formation de thiols. Les résultats mentionnés au tableau 3 mettent, de plus, en évidence le fait que la réaction est pH dépendante. En effet, si l'on fait réagir dans de l'eau distilée, à chaud, de la cystéine avec du bis(2-furfuryl)disulfure, comme à l'essai n°13, le rendement de formation de thiol varie entre 10 et 59%. Par contre si l'on effectue la même réaction dans de l'eau au pH de 7,6, comme dans l'essai n°14, on obtient un rendement de formation de thiols de 87%.

### Test 5: Formation de thiols naturels

On produit par biotransformation des disulfides.

Pour ce faire, on met en suspension une levure boulangère dans un tampon phosphate à pH 8. contenant un conjugué cystéine-aldéhyde à 20 mM.

On effectue cette biotransformation pendant 48 h, pour la moitié de la préparation en milieu anaérobique et, pour l'autre moitié, en milieu aérobique, de manière à comparer l'effet de l'oxygène sur la formation de 2-furfurylthiol.

On mesure alors le rendement de la réaction. La formation de 2-furfurylthiol est à un rendement optimal de 30-40%, en milieu anaérobique, et à rendement optimal de 10-25%, en milieu aérobique.

Après 144 h d'incubation, on obtient la formation de bis (2-furfuryl) disulfure à un rendement optimal de 24% en milieu anaérobique et à un rendement optimal de 4% en milieu aérobique.

On fait alors réagir, en milieu aqueux, a chaud, le bis(2-furfuryl) disulfure naturel, ainsi préparé 144 h en conditions anaérobiques, avec de l'albumine d'oeuf, de manière à produire, à un rendement de 60%, du 2-furfurylthiol isolé à l'aide d'un appareil de distillation-extraction simultanées, le SDE, à pression atmosphérique.

### Exemple 1

On procède à l'aromatisation de café soluble avec le mélange précurseur d'arômes selon l'invention, de manière à développer une note grillée très prononcée.

Pour ce faire, on fait réagir, à chaud dans 100 mi d'eau à pH 7,5, le mélange selon l'invention, comprenant 100 ppm de bis(2-furfuryl) disulfure et de la β lactoglobuline, avec 1,5 g de café soluble.

On obtient un mélange ayant un goût de café avec une note grillée très prononcée.

### Exemple 2

On prépare un mélange précurseur d'arômes sous forme de poudre, pouvant être aisément utilisé pour donner une note aromatique prononcée aux aliments.

Pour ce faire, on forme un mélange précurseur d'arômes stable en mélangant dans de l'eau 100 ppm de bis(2-furfuryl) disulfure avec de l'albumine. On effectue une lyophilisation du mélange précurseur d'arômes ainsi réalisé.

On obtient ainsi un mélange précurseur d'arômes sous forme de poudre que l'on peut facilement utiliser à sec, en suspension ou en solution, notamment, aux micro-ondes, de manière à ce qu'il développe une note aromatique grillée très prononcée.

### Exemple 3

On prépare un fond de sauce au jus de poulet rôti en y ajoutant le mélange selon l'invention.

Pour ce faire, on prépare un mélange précurseur d'arômes tel que décrit à l'exemple 2 et on l'ajoute à un fond de sauce à raison de 0,35% par rapport au poids sec de ce fond de sauce.

On obtient ainsi un fond de sauce au jus de poulet rôti ayant une note de viande très prononcé.

### Exemple 4

On prépare un hamburger en ajoutant dans le steak de viande hâchée le système aromatique selon l'invention.

Pour ce faire, on prépare un mélange précurseur d'arômes tel que décrit à l'exemple 2 et on l'ajoute dans le steak à raison de 0,45% par rapport au poids sec du steack de viande hâchée.

On dépose le steak ainsi préparé sur un demi pain rond avec une garniture composée de salade, de tamotes et de cornichons coupés en rondelles, puis on superpose l'autre portion du pain rond.

On obtient ainsi un hamburger ayant une note de viande très prononcée, lors de la réchauffe aux micro-ondes.

## Revendications

1. Mélange précurseur d'arômes caractérisé par le fait qu'il génère, lors d'une étape de chauffage une note aromatique due à la formation de thiols.

2. Mélange selon la revendication 1, caractérisé par le fait qu'il est constitué d'un polysulfure et d'une source de soufre non volatile comprenant au moins un groupe sulfhydryle.

3. Mélange selon les revendications 1 et 2, dans lequel le polysulfure est de type R-(S)ₙ-R' où R et R' sont identiques ou différents et n est supérieur ou égal à 2.

4. Mélange selon les revendications 1 à 3, dans lequel le polysulfure est obtenu par biotransformation d'un conjugué cystéine-aldéhyde à l'aide d'une levure boulangère.

5. Mélange selon les revendications 1 à 4, dans lequel la source de soufre non volatile combinée ou non à d'autres constituants, est un acide aminé soufré, un peptide contenant au moins un acide aminé soufré, une protéine contenant au moins un acide aminé soufré et/ou un hydrolysat de protéines contenant au moins un acide aminé soufré.

6. Mélange selon les revendications 1 à 5, dans lequel la formation de thiol est à un rendement de 48-90%, lors d'une étape de chauffage à pH neutre.

7. Mélange selon l'une des revendications 1 à 6, dans lequel la source de soufre non volatile est une ou plusieurs protéines comprenant au moins un acide aminé soufré, de manière à permettre la formation d'une poche hydrophobe emprisonnant un ou plusieurs polysulfures.

8. Mélange selon la revendication 7, caractérisé par le fait qu'on le sèche, de manière à obtenir une poudre aromatique stable.

9. Utilisation du mélange selon l'une des revendications 1 à 8 dans la fabrication d'une composition alimentaire, de manière à favoriser le développement d'une note aromatique rôtie, grillée lorsqu'on chauffe la composition alimentaire.

10. Utilisation selon la revendication 9, dans laquelle on ajoute le mélange aromatique dans une proportion de 0,07-0,50% par rapport au poids sec de la composition alimentaire.
